Europäisches Patentamt

European Patent Office

Office européen des brevets

(11) Publication number: **0 390 833 B1**

# EUROPEAN PATENT SPECIFICATION

(45) Date of publication of patent specification: **10.02.93**    (51) Int. Cl.5: **C07C 395/00, C07C 391/00**

(21) Application number: **89900285.1**

(22) Date of filing: **02.12.88**

(86) International application number:
**PCT/GB88/01062**

(87) International publication number:
**WO 89/05293 (15.06.89 89/13)**

(54) **METHOD FOR PURIFICATION OF TELLURIUM AND SELENIUM ALKYLS.**

(30) Priority: **04.12.87 GB 8728393**

(43) Date of publication of application:
**10.10.90 Bulletin 90/41**

(45) Publication of the grant of the patent:
**10.02.93 Bulletin 93/06**

(84) Designated Contracting States:
**DE FR GB IT NL**

(56) References cited:

**J. CHEM. SOC., part III, 1951; G.E. COATES, "Trimethylgallium. Part I. The relative stabilities of its coordination compounds with the methyl derivatives of groups VB and VIB, and the thermal decompositions of some trimethylgallium-amine complexes", see pages 2003-2013**

(73) Proprietor: **THE SECRETARY OF STATE FOR DEFENCE IN HER BRITANNIC MAJESTY'S GOVERNMENT OF THE UNITED KINGDOM OF GREAT BRITAIN AND NORTHERN IRELAND; Whitehall**
**London SW1A 2HB(GB)**

(72) Inventor: **MULLIN, John, Brian**
**The Hoo Brockhill Road**
**West Malvern Worcestershire WR14 4DL(GB)**
Inventor: **COLE-HAMILTON, David, John**
**St Rule Boarhills, by St Andrews**
**Fife KY16 9ST(GB)**
Inventor: **SHENAI-KHATKHATE, Deodatta, Vinayak**
**39 Fife Park St Andrews**
**Fife KY16 5PP(GB)**
Inventor: **WEBB, Paul**
**38 Market Gate South Crail**
**Fife KY10 3TL(GB)**

EP 0 390 833 B1

J. CHEM. SOC., part I, 1934; F. CHALLENGER et al, "The production of organo-metalloidal compounds by microorganisms. Part II. Dimethyl selenide", see pages 68-71

J. CHEM. SOC., Part 1, 1938; F. CARR et al, "The mercuric halides of dimethyl telluride", page 282

AUSTRALIAN JOURNAL OF CHEMISTRY, vol. 26, no. 11, November 1973; J. E. FERGUSSON et al, "A spectroscopic study of sulfide, selenide, and telluride complexes of palladium (II) and mercury (II)", pages 2615-2622

JOURNAL OF ORGANOMETALLIC CHEMISTRY, vol. 286, April 1985, R. K. CHANDRA et al, "Synthesis, characterization and crystal structure of polymeric chloro(diethyl telluride)copper(I),(Et2Te.CuCl)", pages 121-128

J. INDIAN CHEM. SOC., vol. 53, December 1976; B. SINGH et al, "Studies on metal-sulfur and metal-selenium stretching modes in the complexes of zinc(II), cadmium (II) and platinum(II) with dibenzyl sulfide and dibenzyl selenide", pages 1200-1203

(74) Representative: **Beckham, Robert William Defence Research Agency Intellectual Property Department DRA Farnborogh Farnborough, Hants. GU14 6TD(GB)**

**Description**

This invention relates to methods for the purification of tellurium and selenium alkyls, particularly the dialkyls of these metals.

Tellurium and selenium alkyls are important compounds used in the semiconductor industry, for example in the preparation of cadmium mercury telluride (CMT) used in opto-electronic applications. In many such uses the alkyls are decomposed, usually thermally to form the element, which may itself be deposited on a substrate, or combined with other elements in compounds. It is a very important requirement of such alkyls that they be extremely pure, as the presence of even minute quantities of impurities eg on a ppm level can have a detrimental effect on the properties of the semiconductor.

Present methods of purification of tellurium and selenium alkyls generally involve fractional distillation and are unable to easily reach the abovementioned levels of purity. Another known method of purifying metal alkyls involves the formation of an adduct that has properties which allow it to be decomposed in such a way that the pure alkyl is formed as a decomposition product.

The use of adducts in this way to purify tellurium and selenium alkyls is as yet unknown, although some work has been done to investigate various adducts of these alkyls. For example G E Coates, J Chem Soc (1951) pp 2003-2013, Challenger & North, J Chem Soc (1934) 1 pp 68-71, Carr & Pearson, J Chem Soc (1938) p 282, and Ferguson & Loh, Austral. J Chem (1973) 26 pp 2615-22 all mention that these adducts dissociate with relative ease, but none have been previously known to dissociate in such a way as to yield the pure alkyls.

The present invention seeks to overcome the problems of prior art methods of purification of tellurium and selenium alkyls by novel routes using adducts, some of which are themseles novel.

According to the present invention, there is provided a method of purification of a tellurium or selenium alkyl, which is characterised by forming an adduct of the alkyl with at least one compound of a Group 11 or 12 metal (Cu, Ag, Au, Zn, Cd, Hg) and thermally dissociating the said adduct to yield the alkyl. Preferably the at least one compound of a group 11 or 12 metal is an inorganic compound.

The method is suitable for the purification of all tellurium and selenium alkyls, but is particularly suitable for the metal dialkyls. The alkyl group may be straight or branched chain, and is preferably a lower alkyl eg containing up to 4 carbon atoms. These lower alkyls are most useful for semiconductor production.

Preferred Group 11 or 12 metals are cadmium, mercury and copper. The compound is preferably a halide or nitrate, most preferably halides, these being bromides, chlorides and iodides. The most preferred compounts are therefore: $CuI$, $CdI_2$, $CdBr_2$, $HgCl_2$, $Cu_2I_2$, $HgI_2$.

Preferably the method is carried out in the following way:

(i) A solution of the alkyl in a suitable solvent is prepared, and this is added to a solution of the metal compound in a suitable solvent, preferably an organic solvent.

Preferred solvents in both cases are polar aliphatic organic solvents containing 1 to 10 carbon atoms, preferably 1 to 5. It is desirable to use a solvent for the alkyl that is also capable of dissolving the metal compound.

Suitable sovents for the alkyl include ethers, such as THF but especially diethyl ether; alcohols, especially ethanol: and nitriles especially acetonitrile These solvents are also suitable for the metal compound.

Typically the alkyl is dissolved in an ether and is added at an appropriate rate to a solution of the metal compound in an alcohol. Preferably the amount of alkyl and metal compound used are in approximately stoichiometric ratios.

(ii) When reaction appears to be complete, excess solvents may be separated by filtration from any precipitated product, or alternately or additionally the bulk of the solvent may be removed by evaporation.

(iii) The solid adduct product is washed with an appropriate solvent, eg the ether used in the reaction, and excess volatiles removed in vacuo.

(iv) The adduct is thermally dissociated in vacuo to yield the pure alkyl which may be collected in a cooled receiver. Suitable dissociation temperatures will depend upon the adduct and are easily determined experimentally. Generally temperatures in the range 80° - 150°C are suitable.

It is preferred that the preparation and subsequent manipulation of the adduct is performed under an inert atmosphere such as nitrogen.

Appropriate conditions for the preparation of any particular tellurium or selenium alkyl can be easily determined experimentally generally following the procedure outlines above. Alkyls purified by the process of the invention are in many instances in a state of purity suitable for their use directly in the preparation of semiconductors, for example the epitaxial deposit of CMT.

The invention will now be described by way of example only.

Table 1 below lists various adducts which were prepared using the method of the two preparative examples and some of their characteristics. Table 2 below lists the microanalytical data for the various adducts.

Certain of the adducts described below are novel, ie:

$((C_2H_5)_2Te)_2 CdI_2$

$(C_2H_5)_2Te(CdBr_2)_2$

$((C_2H_5)_2Te)_2 CdBr_2$

$((iso\text{-}C_3H_7)_2Te)_2 CdI_2$

$((iso\text{-}C_3H_7)_2Te)_2 CdBr_2$

$(iso\text{-}C_3H_7)_2Te.CdI_2$

Table 1: Adducts for the purification of dialkyltellurium compounds

| Adduct employed | Thermal behaviour (°C) | | % Yield of dialkyltellurium compounds |
|---|---|---|---|
| | melts | dissociates | |
| $(Et_2Te)_2 \cdot CdI_2$ | ~40 | 90 | 37 |
| $Et_2Te \cdot (CdBr_2)_2$ | – | <80 | 67 |
| $Et_2Te \cdot HgCl_2$ | ~100 | ~100–120 | 65 |
| $(Et_2Te \cdot CuI)_n$ | – | <120 | 47 |
| $Et_2Te \cdot HgI_2$ | 45 | <140 | 50 |
| $iPr_2Te \cdot CdI_2$ | ~40 | <130 | 51 |
| $(iPr_2Te)_2 \cdot CdBr_2$ | – | <125 | 66 |
| $(iPr_2Te)_2 \cdot CdI_2$ | – | <125 | 53 |

n is variable and the compound maybe polymeric

4

EP 0 390 833 B1

## Table 2: Preparation of Adducts of $R_2Te$ with Group 11 & 12 Metal compounds

| Dialkyltellurium ($R_2Te$) | Metal Compd. $MX_n$ | Solvent Employed[*] | $R_2Te : MX_n$ ratio in the adduct | Analysis (%) found (theoretical) C | H |
|---|---|---|---|---|---|
| Diethyltellurium ($C_2H_5)_2Te$ | $CdI_2$ | Ethanol | 2:1 | 12.2 (13.0) | 2.5 (2.7) |
| | $CdBr_2$ | Ethanol | 1:2 | 5.7 ( 6.5) | 1.1 (1.3) |
| | $CdBr_2$ | Ethanol | 2:1 | 14.2 (14.9) | 3.1 (2.8) |
| | $HgCl_2$ | Diethylether | 1:1 | 10.4 (10.5) | 2.2 (2.2) |
| | $HgI_2$ | Ethanol | 2:1 | 11.9 (11.6) | 2.2 (2.6) |
| | $Cu_2I_2$ | Acetonitrile | 1:1 | 12.7 (12.7) | 2.6 (2.6) |
| Di-isopropyltellurium $[(CH_3)_2CH]_2Te$ | $CdI_2$ | Ethanol | 2:1 | 18.4 (18.1) | 3.6 (3.5) |
| | $CdBr_2$ | Ethanol | 2:1 | 14.7 (14.8) | 2.8 (2.9) |
| $((CH_3)_2CH)_2Te$ | $CdI_2$ | Ethanol | 1:1 | 12.4 (12.8) | 2.4 (2.6) |

[*] In each case, an ethereal solution of $R_2Te$ was added to the metal halide in the solvent shown.

**Preparative Example 1**

**Purification of diethyltellurium using cadmium iodide**

A solution of diethyltellurium (25.5 g, 0.1373 gmol) in diethylether (50 cm³) was added dropwise to a solution of cadmium iodide (24.1 g, 0.065 gmol) in ethanol (350 cm$^3$) to obtain a white precipitate which dissolved on warming to 50°C. The reaction mixture was cooled and stirred at room temperature for 30 minutes. The volume was reduced to ca 100 cm$^3$ *in vacuo* and the product was obtained as white crystals on cooling to -30°C overnight. The crystals were isolated from the reaction mixture by filtration under a nitrogen atmosphere and by successive washings with petroleum ether cooled to 4°C. The crystals were pumped to remove any volatile impurities for 1h at room temperature.

Microanalytical data suggested that the product had a limiting empirical formula (Et$_2$Te)$_2$CdI$_2$.

The pure adduct, (Et$_2$Te)$_2$CdI$_2$ (34.95 g, 0.0473 gmol) was then heated to 100°C. Pure diethyltellurium distilled and was collected *in vacuo* in a cold trap (11.00 g, 0.0592 gmol, 62.52% yield). The adduct was found to dissociate between 85-90°C.

Preparative Example 2

**Purification of diethyltellurium using Copper(I) iodide**

A solution of diethyltellurium (6.6 g, 0.0355 gmol) in diethylether (25 cm$^3$) was added dropwise to a solution of copper(I) iodide (3.5 g, 0.0183 gmol) in acetonitrile (50 cm$^3$). A pale pink microcrystalline precipitate formed during the addition. The excess diethyltellurium and acetonitrile were removed by filtration under a nitrogen atmosphere and by successive washings with petroleum ether cooled to 4°C. The adduct obtained was pumped for 1h at room temperature to remove any volatile impurities.

Microanalytical studies revealed a limiting empirical formula (Et$_2$Te)CuI.

The adduct, (Et$_2$Te)CuI was then heated to 150°C *in vacuo*. Pure diethyltellurium distilled and was collected in a cold trap. No sublimation of the adduct was found to occur up to 150°C. The dissociation of the adduct was found to occur between 110-130°C.

Preparative Example 3

Purification of di-isopropyltellurium using cadmium iodide

A solution of di-isopropyl tellurium (26.34g, 0.1253 g mol.) in diethyl ether (150 cm$^3$) was added dropwise to a solution of cadmium iodide (22.50g, 0.0614 g mol.) in ethanol (200 cm$^3$). The reaction mixture was cooled to -30°C overnight and the product obtained as white needle crystals. The crystals were isolated from the reaction mixture by filtration under a nitrogen atmosphere and by successive washings with petroleum ether, pre-cooled to 4°C. The crystals were pumped for 1 hour at room temperature to remove any volatile impurities. Overall yield 25.14g (51.47%). Microanalysis of the crystals suggested that the product had a limiting empirical formula (iPrTe)$_2$(CdI$_2$).

The pure adduct (iPrTe)$_2$(CdI$_2$), (25.14g, 0.0316 g mol.) was heated to about 175°C. Pure di-isopropyl tellurium distilled and was collected in vacuo in a cold trap (7.17g, 0.0335 g mol., 53% yield). The adduct was found to dissociate between 125°C-135°C.

Preparative Example 4

Purification of diethyltellurium using Cadmium Bromide

A solution of diethyltellurium (23.32g, 0.1256 g mol) in diethylether (150 cm$^3$) was added dropwise to a solution of cadmium bromide (CdBr$_2$, 4H$_2$O, 88.62g, 0.2574 g mol) in ethanol (200 cm$^3$). A yellowish white microcrystalline precipitate formed during the addition, which was found to redissolve on warming the reaction mixture to 50°C. The solution was cooled and stirred at room temperature for 30 minutes. The colume was then reduced to ca 200 cm$^3$ in vacuo and the product was obtained as white crystals on cooling to -30°C overnight. The crystals were isolated from the reaction mixture by filtration under a nitrogen atmosphere and by successive washings with petroleum ether pre-cooled to 4°C. The crystals were pumped for 1 hour at room temperature to remove any volatile impurities. Overall yield was 93.6% (85.92g).

Microanalysis of the crystals suggested that the product had a limiting empirical formula (Et$_2$Te)-(CdBr$_2$)$_2$.

The pure adduct, (Et$_2$Te)(CdBr$_2$)$_2$, (85.92g, 0.092 g mol) was heated to 140°C. Pure diethyltellurium distilled and was collected in vacuo in a cold trap (14.66g, 0.0789 g mol, 85.85% yield). The adduct was found to dissociate between 80-90°C.

### Preparative Example 5

Purification of diethyltellurium using Mercury (II) Iodide

A solution of diethyltellurium (4.78g 0.025 g mol) was added dropwise to a red solution of mercury (II) iodide (6.16g, 0.013 g mol) in ethanol ( 250 cm$^3$) to obtain a pale yellow crystalline product. The product was isolated from the reaction mixture by filtration under a nitrogen atmosphere and by successive washings with petroleum ether pre-cooled to 4°C. The crystals were pumped to remove any volatile impurities for 1 hour at room temperature.

Microanalytical data suggested that the product had a limiting emperical formula $(Et_2Te)_2 HgI_2$.

The pure adduct $(EtTe)_2 HgI_2$ (5-90g, 7.146 m mol) was then heated to 150°C. Pure diethyl tellurium distilled and was collected in vacuo in a cold trap maintained at -196°C (1.22g, 6.573 m mol, 46.99% yield). The adduct was found to dissociate between 120-140°C.

### Preparative Example 6

Purification of diethyltellurium using Mercury II Chloride

A solution of diethyltellurium (5.71g, 0.0307 g mol) was added dropwise to a solution of mercury (II) Chloride (7.39g, 0.0272 g mol) in diethyl ether (250 cm$^3$) to obtain a pale yellow crystalline product. The product was isolated from the reaction mixture by filtration under a nitrogen atmosphere and by siccessive washings with petroleum ether precooled to 4°C. The crystals were pumped to remove any votalite impurities for 1 hour at room temperature.

Microanalytical data suggested that the product had a limiting empirical formula $Et_2Te HgCl_2$.

The pure adduct $Et_2T_e HgCl_2$ (11.65g, 0.0254 g mol) was then heated to 120°C. Pure diethyltellurium distilled and was collected in vacuo in a cold trap maintained at the liquid nitrogen temperature. The adduct was found to melt at ca 100°C and dissociate between 100-120°C.

### Preparative Example 7

Purification of di-isopropyltellurium using cadmium iodide

A solution of di-isopropyltellurium (61.48 g. 0.288 g mol) in diethyl ether (50 cm$^3$) was added dropwise to a solution of cadmium iodide (130.58 g, 0.3565 g mol) in ethanol (ca 250 cm$^3$) to afford a clear orange solution. The reaction mixture was cooled to -30°C overnight and the product was obtained as white needle shaped crystals. These crystals were isolated from the reaction mixture by filtration under a nitrogen atmeosphere, and by successive washings with petroleum ether precooled to 4°C. The crystals were pumped for 1 hour at room temperature to remove any volatile impurities.

Microanalysis of the product suggested that the product had a limiting composition $(i-C_3H_7)_2Te.CdI_2$.

The pure adduct (192.06 g, 0.331 mol) was heated to ca. 130°C Pure di-isopropyltellurium distilled and was collected in vacuo in a cold trap maintained at -196°C. Yield was 31.26g, 50.81% based on the starting $(i-C_3H_7)_2Te$.

### Preparative Example 8

Purification of di-isopropyltellurium using cadmium bromide

A solution of di-isopropyltellurium (4.62 g, 0.0216 gmol) in ethanol (50 cm$^3$) was added dropwise to a solution of cadmium bromide $(CdBr_2.4H_2O$, 9.91 g) in ethanol (50 cm$^3$) A yellow-white microcrystalline precipitate formed during the addition The product was isolated from the reaction mixture by filtration under a nitrogen atmosphere and by successive washings with petroleum ether pre-cooled to 4°C. The crystls were pumped for 1 hour at room temperature to remove any volatile impurities.

Microanalysis of the crystals suggested that the product had a limiting composition $CdBr_2((i-C_3H_7)_2Te)_2$.

The pure adduct (2.5 g) was heated to ca.125°C Pure di-isopropyltellurium distilled and was collected in vacuo in a cold trap maintained at -196°C. Yield was 1.oo g. 65.56 % based on the weight of the adduct.

**Claims**

7

1. A method of purification of a tellurium or selenium alkyl characterised by forming an adduct of the alkyl with at least one compound of a group 11 or 12 metal (Cu, Ag, Au, Zn, Cd, Hg) and thermally dissociating the said adduct to yield the alkyl.

2. A method of purification according to claim 1 characterised in that the tellurium or selenium alkyl is a dialkyl.

3. A method of purification according to claim 1 or 2 characterised in that each alkyl group in the tellurium or selenium alkyl contains 1 to 4 carbon atoms.

4. A method of purification according to any one of claims 1, 2 or 3 characterised in that the alkyl is a tellurium alkyl.

5. A method according to claim 1 characterised in that the compound of a group 11 or 12 metal is a halide or nitrate thereof.

6. A method of purification according to claim 5 characterised in that the metal compound is a chloride, bromide or iodide.

7. A method of purification according to claim 6 characterised in that the at least one compound of a group 11 or 12 metal is selected from $CuI$, $CdI_2$, $CdBr_2$, $HgCl_2$ $Cu_2I_2$ and $HgI_2$.

8. A method of purification according to claim 1 characterised in that the adduct is formed by reaction between the alkyl and the metal compound in an organic solvent.

9. A method according to claim 8 characterized in that the solvent is selected from ethers, alcohols and nitriles and mixtures thereof.

10. A method of purification according to claim 1 characterised in that the adduct is dissociated at 80°C - 150°C.

11. A method of purification according to claim 1 characterised in that diethyl tellurium is purified by forming an adduct with a compound selected from $CdI_2$, $CdBr_2$, $HgCl_2$, $CuI_2$, and $HgI_2$ and then thermally dissociating the adduct and collecting the alkyl.

12. A method of purification according to claim 1 characterised in that diisopropyl tellurium is purified by forming an adduct with a compound selected from $CdI_2$ and $CdBr_2$ and then thermally dissociating the adduct and collecting the alkyl.

13. An adduct of dialkyl tellurium having a formula selected from following:
$((C_2H_5)_2Te)_2CdI_2$
$(C_2H_5)_2Te(CdBr_2)_2$
$((C_2H_5)_2Te)_2CdBr_2$
$((i-C_3H_7)_2Te)_2CdI_2$
$((i-C_3H_7)_2Te)_2CdBr_2$
$(i-C_3H_7)_2TeCdI_2$

**Patentansprüche**

1. Verfahren zur Reinigung eines Tellur- oder Selenalkyls, gekennzeichnet durch Bildung eines Addukts des Alkyls mit mindestens einer Verbindung eines Metalls der Gruppe 11 oder 12 (Cu, Ag, Au, Zn, Cd, Hg) und thermisches Dissoziieren dieses Addukts, um das Alkyl zu erhalten.

2. Reinigungsverfahren nach Anspruch 1, dadurch gekennzeichnet, daß das Tellur- oder Selenalkyl ein Dialkyl ist.

3. Reinigungsverfahren nach Anspruch 1 oder 2, dadurch gekennzeichnet, daß jede Alkylgruppe in dem Tellur- oder Selenalkyl 1 bis 4 Kohlenstoffatome enthält.

4.   Reinigungsverfahren nach einem der Ansprüche 1, 2 oder 3, dadurch gekennzeichnet, daß das Alkyl ein Telluralkyl ist.

5.   Verfahren nach Anspruch 1, dadurch gekennzeichnet, daß die Verbindung eines Metalls der Gruppe 11 oder 12 ein Halogenid oder ein Nitrat hiervon ist.

6.   Reinigungsverfahren nach Anspruch 5, dadurch gekennzeichnet, daß die Metallverbindung ein Chlorid, Bromid oder Jodid ist.

7.   Reinigungsverfahren nach Anspruch 6, dadurch gekennzeichnet, daß die mindestens eine Verbindung eines Metalls der Gruppe 11 oder 12 aus CuI, $CdI_2$, $CdBr_2$, $HgCl_2$, $Cu_2I_2$ und $HgI_2$ ausgewählt ist.

8.   Reinigungsverfahren nach Anspruch 1, dadurch gekennzeichnet, daß das Addukt durch Reaktion zwischen dem Alkyl und der Metallverbindung in einem organischen Lösungsmittel gebildet wird.

9.   Verfahren nach Anspruch 8, dadurch gekennzeichnet, daß das Lösungsmittel aus Äthern, Alkoholen und Nitrilen und Gemischen hiervon ausgewählt ist.

10.  Reinigungsverfahren nach Anspruch 1, dadurch gekennzeichnet, daß das Addukt bei 80°C bis 150°C dissoziiert wird.

11.  Reinigungsverfahren nach Anspruch 1, dadurch gekennzeichnet, daß das Diäthyltellur durch Bildung eines Addukts mit einer Verbindung, die aus $CdI_2$, $CdBr_2$, $HgCl_2$, $CuI_2$ und $HgI_2$ ausgewählt ist, und anschließendes thermisches Dissoziieren des Addukts und Sammeln des Alkyls gereinigt wird.

12.  Reinigungsverfahren nach Anspruch 1, dadurch gekennzeichnet, daß das Isopropyltellur durch Bildung eines Addukts mit einer Verbindung, die aus $CdI_2$ und $CdBr_2$ ausgewählt ist, und anschließendes thermisches Dissoziieren des Addukts und Sammeln des Alkyls gereinigt wird.

13.  Dialkyltellur-Addukt mit einer aus nachstehendem ausgewählten Formel:
$((C_2H_5)_2Te)_2CdI_2$
$(C_2H_5)_2Te(CdBr_2)_2$
$((C_2H_5)_2Te)_2CdBr_2$
$((i-C_3H_2)_2Te)_2CdI_2$
$((i-C_3H_7)_2Te)_2CdBr_2$
$(i-C_3H_7)_2TeCdI_2$

**Revendications**

1.   Un procédé de purification d'un tellure- ou sélénium-alkyle, caractérisé par la formation d'un produit d'addition de l'alkyle avec au moins un composé d'un métal du groupe 11 ou 12 (Cu, Ag, Au, Zn, Cd, Hg) et la dissociation thermique dudit produit d'addition pour former l'alkyle.

2.   Un procédé de purification selon la revendication 1, caractérisé en ce que le tellure- ou sélénium-alkyle est un dialkyle.

3.   Un procédé de purification selon la revendication 1 ou 2, caractérisé en ce que chaque groupe alkyle dans le tellure- ou sélénium-alkyle contient 1 à 4 atomes de carbone.

4.   Un procédé de purification selon l'une quelconque des revendications 1, 2 ou 3, caractérisé en ce que l'alkyle est un tellure-alkyle.

5.   Un procédé selon la revendication 1, caractérisé en ce que le composé d'un métal du groupe 11 ou 12 est un halogénure ou un nitrate de celui-ci.

6.   Un procédé de purification selon la revendication 5, caractérisé en ce que le composé métallique est un chlorure, bromure ou iodure.

**7.** Un procédé de purification selon la revendication 6, caractérisé en ce que le ou les composés d'un métal du groupe 11 ou 12 est/sont choisi(s) parmi CuI, $CdI_2$, $CdBr_2$, $HgCl_2$, $Cu_2I_2$ et $HgI_2$.

**8.** Un procédé de purification selon la revendication 1, caractérisé en ce que le produit d'addition est formé par réaction entre l'alkyle et le composé métallique dans un solvant organique.

**9.** Un procédé selon la revendication 8, caractérisé en ce que le solvant est choisi parmi les éthers, les alcools, les nitriles et leurs mélanges.

**10.** Un procédé de purification selon la revendication 1, caractérisé en ce que le composé d'addition est dissocié entre 80°C et 150°C.

**11.** Un procédé de purification selon la revendication 1, caractérisé en ce que l'on purifie le tellure-diéthyle par formation d'un produit d'addition avec un composé choisi parmi $CdI_2$, $CdBr_2$, $HgCl_2$, $CuI_2$ et $HgI_2$, puis dissociation thermique du produit d'addition et récolte de l'alkyle.

**12.** Un procédé de purification selon la revendication 1, caractérisé en ce que l'on purifie le tellure-diisopropyle par formation d'un produit d'addition avec un composé choisi parmi $CdI_2$ et $CdBr_2$, puis dissociation thermique du produit d'addition et récolte de l'alkyle.

**13.** Un produit d'addition de tellure-dialkyle ayant une formule choisie parmi les suivantes :
$((C_2H_5)_2Te)_2CdI_2$
$(C_2H_5)_2Te(CdBr_2)_2$
$((C_2H_5)_2Te)_2CdBr_2$
$((i\text{-}C_3H_7)_2Te)_2CdI_2$
$((i\text{-}C_3H_7)_2Te)_2CdBr_2$
$(i\text{-}C_3H_7)_2TeCdI_2$